Europäisches Patentamt

**European Patent Office**

Office européen des brevets

(19)

(11) Numéro de publication : **0 112 319**
**B1**

(12)

# FASCICULE DE BREVET EUROPÉEN

(45) Date de publication du fascicule du brevet :
01.10.86

(21) Numéro de dépôt : 83870117.5

(22) Date de dépôt : 09.11.83

(51) Int. Cl.⁴ : **A 61 K  6/06**, A 61 K  9/22,
**A 61 L 15/06**

(54) Matériaux bioréactifs.

(30) Priorité : 02.12.82 BE 209635

(43) Date de publication de la demande :
27.06.84 Bulletin 84/26

(45) Mention de la délivrance du brevet :
01.10.86 Bulletin 86/40

(84) Etats contractants désignés :
AT BE CH DE FR GB IT LI LU NL SE

(56) Documents cités :
EP-A- 0 023 013
DE-A- 2 130 984
FR-A- 2 297 887
US-A- 2 980 998
US-A- 4 120 729
CHEMICAL ABSTRACTS, vol. 94, 1981, page 406, no.
109391v, Columbus, Ohio, US

(73) Titulaire : **Région Wallonne représentée par l'Exécutif Régional Wallon**
**11, Boulevard de l'Empereur**
**B-1000 Bruxelles (BE)**

(72) Inventeur : **Richez, Raymond Michel**
**rue des Américains 59**
**B7020 Mons (BE)**

(74) Mandataire : **Blanchart, André**
**MINISTERE DE LA REGION WALLONE Direction d'Administration de l'Energie et des Technologies nouvelles Service des Technologies nouvelles Avenue Prince de Liège, 7**
**B-5100 Namur (BE)**

## Description

La présente invention a pour objet des nouveaux matériaux bioréactifs utilisés notamment pour les implants et les prothèses sur hommes ou animaux dans les domaines médicaux, dentaires et vétérinaires.

Par matériaux bioréactifs, suivant la présente invention, on comprend des matériaux résultant de la fusion de mélanges d'oxydes ou de sels ou de tout autre précurseur métallique et qui sont susceptibles de réagir biochimiquement avec les tissus vivants et plus particulièrement les tissus osseux.

Les verres et vitrocéramiques biologiquement actifs sont connus depuis de nombreuses années. Ainsi des compositions comprenant de 40 à 60 % de SiO$_2$ et B$_2$O$_3$, 25 % de Na$_2$O, 25% de CaO et quelques pourcents de P$_2$O$_5$ ont fait l'objet de nombreuses expérimentations. Dans le cas de telles compositions, l'accrochage avec l'os est tributaire de la formation en surface du matériau d'une structure microporeuse développant une grande surface spécifique, par exemple un gel de silice, protégé par une couche riche en ions Ca et P. La diffusion sélective des ions sodium, calcium et phosphore, la dissolution partielle du réseau du matériau sont les principales étapes qui conduisent à cette structure superficielle induisant la minéralisation de l'os à sa surface.

Suivant les brevets US 2301488 et 2243915, des matériaux formés d'une phase vitreuse et d'une phase cristalline telle que l'hydroxyapatite ou des autres composés du même groupe cristallin sont basés sur le principe selon lequel la présence de phases cristallines hexagonales dans la phase vitreuse favorise la croissance épitaxique de l'apatite dans le matériau. « Suivant le EP-A-23013, les particules de poudres de verre constituées de calcium aluminium fluorosilicate sont traitées superficiellement de manière à appauvrir la couche superficielle en Ca.

Contrairement à la présente demande, la porosité de cette couche est obtenue par un traitement ultérieur ».

Suivant l'art antérieur un même principe réactionnel à la surface du matériau en contact avec les tissus vivants conduit toujours à la formation d'un milieu développant une grande surface spécifique comme par exemple un gel de silice.

Ces divers matériaux ont un pouvoir ostéogénique lorsqu'ils sont au contact d'un milieu physiologique. Néanmoins, des expériences réalisées sur moutons dont les résultats sont exposés dans J. of. Biomedic. Mater. Res. Vol. 12, 57-65 (1978) ont montré que la structure superficielle développée par les matériaux classiques n'assure pas une tenue mécanique à long terme des implants qu'ils recouvrent. Dans un milieu corrosif tel que le milieu physiologique, cette structure superficielle se détruit et provoque une corrosion trop importante de l'implant.

La présente invention a pour but de pallier ces inconvénients en agissant sur la réactivité de surface du matériau de manière à augmenter la résistance à la corrosion des couches superficielles. En conséquence leur résistance mécanique à long terme est améliorée tout en gardant le même pouvoir ostéogénique que les matériaux connus jusqu'à présent.

La présente invention a pour objet des nouveaux matériaux bioréactifs développant en leur surface au contact des tissus vivants une couche silico-aluminium qui se forme après dissolution sélective d'ions dans le milieu environnant. De tels matériaux bioréactifs qui n'ont jamais été synthétisés jusqu'à présent sont obtenus par le choix en proportions définies des divers oxydes métalliques les constituant.

Les matériaux bioréactifs, suivant la présente invention sont caractérisés en ce qu'ils sont obtenus par fusion d'un mélange de 20 à 50 % en poids de SiO$_2$, 15 à 25 % en poids de CaO, 5 à 10 % en poids de P$_2$O$_5$, 15 à 25 % en poids de Na$_2$O et 3 à 15 % en poids de Al$_2$O$_3$ ou de leurs précurseurs métalliques donnant par fusion des quantités équivalentes en oxydes.

Par fusion, on entend tout procédé qui permet d'obtenir à une température supérieure à 500 °C une structure vitreuse. Le mélange avant fusion peut être obtenu par les techniques connues comme par exemple le mélange des poudres ou les techniques indirectes comme par exemple la méthode des gels.

Par précurseur métallique, on entend tout composé métallique susceptible de donner des oxydes par fusion. Par exemple, les hydroxydes, carbonates et nitrates peuvent être utilisés suivant la présente invention en quantité équivalente de telle manière que l'on obtienne par fusion les quantités désirées en oxydes.

Les compositions bioréactives qui font l'objet de la présente invention doivent toujours contenir un taux minimum d'ions formateurs de réseaux : SiO$_2$, Al$_2$O$_3$ qui garantissent la formation au contact des tissus osseux d'une couche silico-alumineuse et la stabilité chimique à long terme du matériau.

La teneur en SiO$_2$ doit être supérieure à 20 % parce qu'il est l'ion formateur principal du réseau du verre. Une teneur supérieure à 50 % de SiO$_2$ diminue fortement la réactivité du matériau bioréactif.

La présence d'oxyde d'aluminium renforce la résistance à la corrosion des structures superficielles du matériau bioréactif. Il a été constaté que l'Al$_2$O$_3$ doit être présent en une quantité d'au moins environ 3 % en poids pour pouvoir jouer ce rôle. Une forte teneur en alumine au-delà de 15 % en poids provoque une série de phénomènes difficilement contrôlables durant la préparation, notamment la dévitrification qui modifie les propriétés des matériaux bioréactifs.

Les quantités de P$_2$O$_5$ utilisées dans les

compositions pour l'obtention des matériaux bioréactifs sont comprises entre 5 et 10 % en poids. Cet élément contribue à la formation de l'os à la surface du matériau mais une augmentation de la limite supérieur n'apporte aucun avantage parti-culier.

Les teneurs en ions alcalins et alcalino-terreux déterminent les réactivités à court et à moyen terme du matériau.

Ces teneurs doivent donc être judicieusement choisies en fonction de différents paramètres d'utilisation comme par exemple la vascularisation du milieu physiologique. Elles sont comprises entre 15 et 25 % en poids de CaO et de $Na_2O$ ; le CaO pouvant être substitué par du MgO et/ou du $CaF_2$ et le $Na_2O$ par du $K_2O$ et/ou du $Li_2O$.

Les expériences réalisées *in vivo* ont montré que la présence de CaO et $P_2O_5$ dans les maté-riaux bioréactifs favorise la repousse osseuse à la surface de celui-ci. De plus, le rapport des teneurs en CaO et $P_2O_5$ est choisi de telle manière que ce phénomène soit optimal. Préférentiellement ce rapport est de 3.

Suivant la présente invention, les nouvelles compositions de matériaux bioréactifs peuvent comprendre en plus 5 à 25 % en poids de $B_2O_3$. Ce pourcentage de $B_2O_3$ peut être réglé de manière à diminuer la température de fusion de l'ensemble des constituants et faciliter les processus de préparation du matériau.

Il est à remarquer que l'adjonction de $B_2O_3$ a une influence sur les caractéristiques physiologiques du matériau final. Selon les utilisations envisagées, des caractéristiques physiques telles que le coefficient de dilatation thermique et l'évolution de la viscosité en fonction de la température peuvent être ajustées en faisant varier la quantité de $B_2O_3$ dans le matériau dans les limites revendiquées.

La couche silico-alumineuse qui se forme en présence des tissus vivants constitue un milieu qui possède des propriétés catalytiques particulièrement intéressantes. En outre, elle possède une résistance à la corrosion en milieu alcalin beaucoup plus grande que le gel de silice.

Il est de cette manière possible de mieux contrôler le comportement à long terme de ce matériau.

Des expériences ont montré que cette couche silico-alumineuse était stable dans des milieux dont le Ph vaut 11 et que dans ces conditions il n'était pas nécessaire qu'elle soit protégée par une couche Ca-P pour garder une épaisseur constante. Ce fait est une illustration de l'effet bénéfique de l'adjonction d'ions $Al^{3+}$ dans un réseau silicaté sur la corrosion de celui-ci.

Suivant une réalisation préférentielle de l'invention, les matériaux bioréactifs sont obtenus par fusion d'un mélange de 25 à 35 % en poids de $SiO_2$, de 15 à 25 % en poids de CaO, de 5 à 10 % en poids de $P_2O_5$, de 15 à 25 % en poids de $Na_2O$, de 5 à 12 % en poids d'$Al_2O_3$ et de 5 à 25 % en poids de $B_2O_3$ ou de leurs précurseurs métalliques donnant par fusion des quantités équivalentes en oxydes. « Suivant une autre réalisation préférentielle, une composition identique mais contenant de 10 à 20 % en poids de $B_2O_3$ est utilisée ».

D'autres oxydes métalliques peuvent être éventuellement présents dans les matières premières ou peuvent être ajoutées en quantités faibles aux matières sans sortir du cadre de la présente invention. De tels oxydes jouent un rôle négligeable sur les propriétés du matériau final.

Les nouveaux matériaux bioréactifs faisant l'objet de la présente invention sont obtenus par fusion telle que définie ci-avant ou par tout autre procédé similaire connu. Par exemple, par la méthode des poudres, la fusion peut être réalisée à des températures comprises entre 1 100 et 1 450 °C pendant des temps de 3 à 12 h. Par la méthode des gels, la fusion peut être réalisée à des températures beaucoup plus basses, par exemple légèrement supérieures à 500 °C. Suivant la première méthode, les composants sont par exemple prémélangés dans une jarre rotative pendant quelques heures avant de subir le traitement de fusion. Le mélange fondu est, soit coulé dans des moules de graphite de forme désirée dans lesquels le produit est recuit, ou bien coulé et broyé par exemple en poudre ou en particules de granulométrie désirée.

Tout autre technique de fusion, de moulage ou de coulage, d'injection peut être utilisée pour la fabrication de matériaux bioréactifs. Les nouveaux matériaux bioréactifs trouvent de nombreuses applications notamment dans les domaines médicaux, dentaires et vétérinaires.

Les matériaux bioréactifs faisant l'objet de la présente invention sont utilisés comme matériau de base ou de recouvrement. Par matière de base, on comprend également toute composition obtenue à base des matériaux bioréactifs faisant l'objet de la présente invention et d'autres matériaux, charges et/ou additifs ; ces derniers composés pouvant être des poudres céramiques comme par exemple l'alumine ou l'hydroxyapa-tite. Les matériaux de base peuvent se présenter sous différentes formes par exemple sous forme de poudres, de particules, de flocons et de fibres. Ils peuvent être utilisés pour la fabrication de produits finis les plus divers : par exemple des produits conformés de forme quelconque, des tissus, tricots ou matériaux non tissés trouvant des applications dans les domaines médicaux, dentaires et vétérinaires.

En utilisant des techniques connues par exemple de coulage et de moulage, on peut réaliser à partir de ces nouveaux matériaux bioréactifs tout implant ou prothèse.

Les matériaux bioréactifs faisant l'objet de la présente invention sont utilisés également comme matériau de recouvrement ou d'enduction des implants et/ou prothèses. Ces derniers peuvent être recouverts partiellement ou totalement. Ils se prêtent particulièrement bien au recouvrement de pièces en céramique d'oxyde d'aluminium sans craindre de diffusion des ions $Al^{3+}$ lors de la déposition mais peuvent aussi servir au recouvrement ou à l'enduction de pièces composées d'autres céramiques, de métaux ou de verres. Dans ce type d'applications, on fait usage de toute technique connue de recouvrement, par exemple la déposition par chalumeau de projection plasma.

Les nouveaux matériaux bioréactifs revendiqués trouvent notamment une application intéressante pour la fixation des prothèses de la hanche. Par l'utilisation de ces matériaux bioréactifs, il est possible en utilisant des formes de prothèses autobloquantes de les fixer sans emploi de ciment et d'obtenir ainsi un cimentage biologiquement idéal puisque ces matériaux bioréactifs participent à la repousse du tissu osseux et à la liaison avec ceux-ci.

Les nouveaux matériaux bioréactifs peuvent également trouver de nombreuses applications dans les domaines médicaux autres qu'orthopédiques. A titre d'exemple, citons le remplacement des osselets de l'oreille interne.

Dans le domaine dentaire, les matériaux bioréactifs peuvent être utilisés comme composants d'implants pour la fixation des couronnes dentaires.

Dans le domaine vétérinaire, ils peuvent servir à réaliser par exemple, des prothèses orthopédiques pour chiens et pour chevaux.

Lorsqu'ils sont implantés dans un milieu vivant, ces matériaux bioréactifs subissent à leur surface une séquence de diffusions sélectives chimiques qui conduisent à la formation d'un milieu favorable à la repousse osseuse.

Cette réactivité en milieu physiologique conduit à la formation d'un lien chimique durable et mécaniquement stable avec les os environnants. C'est pourquoi, ces matériaux sont destinés notamment à la fixation de prothèses d'implants en contact avec les os ou avec un milieu physiologique dans lequel l'ostéogenèse est possible.

La présente invention sera mieux comprise à l'aide des exemples non limitatifs décrits ci-après :

Exemple 1

Un matériau bioréactif a été obtenu par fusion à 1 350 ºC pendant 3 heures d'un mélange de 21 % en poids de $SiO_2$, de 24,5 % en poids de CaO et de $Na_2O$, de 6 % en poids de $P_2O_5$, de 9 % en poids d'$Al_2O_3$ et de 15 % en poids de $B_2O_3$. Une composition classique connue comprenant 30 % en poids de $SiO_2$, 15 % en poids de $B_2O_3$, 24,5 % en poids de $Na_2O$ et de CaO et 6 % en poids de $P_2O_5$, ne contenant pas d'$Al_2O_3$ a été utilisée comme composition comparative.

Les avantages apportés par l'invention ont pu être mis en évidence en faisant subir au nouveau matériau des tests de corrosion *in vitro* et *in vivo*.

Une partie des tests *in vitro* a été réalisée sur des poudres de 44 μm à 104 μm obtenues par broyage de la masse coulée et tamisage, dont la distribution granulométrique est contrôlée par sédigraphie. Une autre partie des tests *in vitro* est effectuée sur des plaquettes parallélipédiques préalablement découpées et rodées à l'outil diamanté. Le test consiste à exposer les poudres et les plaquettes à une solution aqueuse tamponnée a pH 7.2 et non tamponnée (eau déminéralisée) pendant des temps variant de 1 jour à 8 semaines.

L'évolution des phases cristallines est suivie par diffraction de rayons X, les analyses chimiques sont réalisées par spectrophotométrie plasma sur les poudres et l'analyse des profils de concentration des différents éléments à la surface des plaquettes a pu être réalisée à l'aide d'une microsonde électronique. Une méthode d'adsorption d'azote permet de suivre l'évolution de la surface spécifique des poudres.

L'étude de la cinétique de corrosion *in vitro* de la composition classique utilisée à titre comparatif et de la nouvelle composition revendiquée a permis de mettre en évidence les différences fondamentales entre les comportements de surface de ces deux matériaux :

La réactivité de surface du nouveau matériau bioréactif est plus grande que celle de la composition classique (diffusion plus rapide des ions modificateurs $Ca^{++}$, $Na^+$).

Dans un premier temps, l'oxide d'aluminium participe à la formation du réseau de la phase vitreuse du matériau bioréactif ;

Le léchage des ions $Ca^{++}$ et $Na^+$ de la surface du nouveau matériau induit la formation d'un gel silico-alumineux développant une surface spécifique de plusieurs $m^2/g$ et particulièrement résistant à la corrosion alcaline. Cette couche silico-alumineuse en surface forme en elle-même une protection bloquant, par sa présence, les phénomènes de diffusion des ions modificateurs.

D'autre part, les tests *in* vivo décrits ci-dessous montrent que le nouveau matériau, par des mécanismes simples, réalise un lien chimique remarquable avec les tissus osseux avec lesquels ils sont en contact.

Des implants ont été préparés à partir des différents oxydes ou différents carbonates correspondants à la composition reprise ci-avant par fusion à des températures de 1 350 ºC pendant 3 heures et par coulée dans des moules parallélipédiques en graphite.

Les pièces ainsi obtenues sont découpées afin d'obtenir des implants de même que ceux testés *in vitro*. Ceux-ci sont polis à la meule diamantée et stérilisés au dioxyde d'éthylène. Les implants sont alors placés dans des rats mâles Sprague Dawley de 150 g. On anesthésie l'animal. Sur la surface intérieure de la patte arrière gauche, on dégage le tibia des muscles environnants, on réalise une fente à travers les cortex latéraux du tibia à l'aide d'un foret électrique et on insère l'implant dans la fente. On referme l'incision par points et agraphes. La fixation avec l'os est évaluée après 30 jours d'implantation. Les tibias sont extraits des animaux sacrifiés et délestés des tissus adhérents. La zone proche de l'implant est examinée à la fois par coupes histologiques réalisées après enlèvement de l'implant hors de l'os récepteur, à la fois par microsonde électronique après lyophilisation. L'examen des implants extraits des animaux sacrifiés après 1 mois montre que l'incision réalisée dans le tibia était complètement comblée par de l'os minéralisé et que des excroissances osseuses sur l'implant fixaient celui-ci de manière parfaite.

Des analyses au travers de l'interface corticale-implant ont montré clairement la barrière silico-alumineuse formée à la surface du nouveau matériau, cette barrière est perméable aux macromolécules de collagène et aux muccopoly-saccharides et sert de milieu catalyseur pour la minéralisation de l'os.

Les mêmes expériences ont été réalisées sur une composition classique. Les résultats montrent clairement que, dans la plupart des cas, ces matériaux subissent une séquence de corrosion qui conduit à moyen terme à la destruction totale du matériau.

Exemple 2

Des implants ont été réalisés à partir d'un alliage de titane recouvert du matériau bioréactif dont la composition est donnée à l'exemple 1.

Des poudres de granulométrie comprises entre 60 et 100 μm ont été synthétisées pour recouvrir sous forme d'une couche de 300 μm les implants.

Les mêmes tests in vitro et in vivo que ceux de l'exemple 1 ont été appliqués sur les implants recouverts du matériau bioréactif.

Les résultats obtenus sont comparables à ceux de l'exemple 1 et les mêmes conclusions sur les qualités ostéogéniques et la résistance à la corrosion du matériau bioréactif peuvent être tirées.

**Revendications**

1. Matériaux bioréactifs caractérisés en ce qu'ils sont obtenus par fusion d'un mélange de 20 à 50 % en poids de $SiO_2$, 15 à 25 % en poids de CaO, 5 à 10 % en poids de $P_2O_5$, 15 à 25 % en poids de $Na_2O$ et 3 à 15 % en poids d'$Al_2O_3$ ou de leurs précurseurs métalliques donnant par fusion des quantités équivalentes en oxydes.

2. Matériaux bioréactifs suivant la revendication 1 caractérisés en ce que le CaO peut être substitué en partie par du MgO et/ou par du $CaF_2$ et le $Na_2O$ par du $K_2O$ et/ou par du $Li_2O$.

3. Matériaux bioréactifs suivant les revendications 1 et 2 caractérisés en ce qu'ils comprennent en plus 5 à 25 % en poids de $B_2O_3$.

4. Matériaux bioréactifs suivant les revendications 1 à 3 caractérisés en ce qu'ils sont obtenus par fusion d'un mélange de 25 à 35 % en poids de $SiO_2$, 15 à 25 % en poids de CaO, 5 à 10 % en poids de $P_2O_5$, 15 à 25 % en poids de $Na_2O$, 5 à 12 % en poids d'$Al_2O_3$ et 5 à 25 % en poids de $B_2O_3$ ou de leurs précurseurs métalliques donnant par fusion des quantités équivalentes en oxydes.

5. Matériaux bioréactifs suivant les revendications 1 à 4 caractérisés en ce qu'ils sont utilisés comme matériaux de base ou comme matériaux de recouvrement des implants et prothèses dans les domaines médicaux, dentaires et vétérinaires.

**Claims**

1. Bioreactive materials, characterised in that they are obtained by fusion of a mixture of from 20 to 50 % by weight of $SiO_2$, 15 to 25 % by weight of CaO, 5 to 10 % by weight of $P_2O_5$, 15 to 25 % by weight of $Na_2O$ and 3 to 15 % by weight of $Al_2O_3$, or of their metallic precursors giving by fusion equivalent quantities of oxides.

2. Bioreactive materials according to Claim 1, characterised in that the CaO can be partly substituted by MgO and/or by $CaF_2$, and the $Na_2O$ by $K_2O$ and/or by $Li_2O$.

3. Bioreactive materials according to Claims 1 and 2, characterised in that they additionally comprise 5 to 25 % by weight of $B_2O_3$.

4. Bioreactive materials according to Claims 1 to 3, characterised in that they are obtained by fusion of a mixture of from 25 to 35 % by weight of $SiO_2$, 15 to 25 % by weight of CaO, 5 to 10 % by weight of $P_2O_5$, 15 to 25 % by weight of $Na_2O$, 5 to 12 % by weight of $Al_2O_3$ and 5 to 25 % by weight of $B_2O_3$, or of their metallic precursors giving by fusion equivalent quantities of oxides.

5. Bioreactive materials according to Claims 1 to 4, characterised in that they are used as base materials or as materials for covering implants and prostheses in the medical, dental and veterinary fields.

**Patentansprüche**

1. Bioreaktive Materialien, dadurch gekennzeichnet, daß sie durch Verschmelzung einer Mischung von 20 bis 50 Gew.-% $SiO_2$, 15 bis 25 Gew.-% CaO, 5 bis 10 Gew.-% $P_2O_5$, 15 bis 25 Gew.-% $Na_2O$ und 3 bis 15 Gew.-% $Al_2O_3$ oder von ihren metallischen Vorläufern, die durch Verschmelzung equivalente Oxidmengen ergeben, erhalten werden.

2. Bioreaktive Materialien nach Anspruch 1, dadurch gekennzeichnet, daß das CaO teilweise durch MgO und/oder $CaF_2$ und das $Na_2O$ durch $K_2O$ und/oder durch $Li_2O$ ersetzt ist.

3. Bioreaktive Materialien nach den Ansprüchen 1 und 2, dadurch gekennzeichnet, daß sie zusätzlich 5 bis 25 Gew.-% $B_2O_3$ umfassen.

4. Bioreaktive Materialien nach den Ansprüchen 1 bis 3, dadurch gekennzeichnet, daß sie durch Verschmelzung einer Mischung von 25 bis 35 Gew.-% $SiO_2$, 15 bis 25 Gew.-% CaO, 5 bis 10 Gew.-% $P_2O_5$, 15 bis 25 Gew.-% $Na_2O$, 5 bis 12 Gew.-% $Al_2O_3$ und 5 bis 25 Gew.-% $B_2O_3$ oder von ihren metallischen Vorläufern, die durch Verschmelzung equivalente Oxidmengen ergeben, erhalten werden.

5. Bioreaktive Materialien nach den Ansprüchen 1 bis 4, dadurch gekennzeichnet, daß sie als Basismaterialien oder als Überzugsmaterialien von Implantaten und Prothesen in den medizinischen, zahnmedizinischen und veterinärmedizinischen Bereichen verwendet werden.